# EUROPEAN PATENT SPECIFICATION

(11) **EP 1 711 151 B1**
(45) Date of publication and mention of the grant of the patent: **18.07.2012**
(21) Application number: 04800306.5
(22) Date of filing: 11.11.2004
(51) Int. Cl.: A61F 13/471

(54) **ACCORDION FOLDED INCONTINENCE DEVICE**
WIE EIN AKKORDEON GEFALTETE INKONTINENZVORRICHTUNG
DISPOSITIF POUR INCONTINENCE PLIE EN ACCORDEON

(30) Priority: 11.11.2003 SE 0302967
(43) Date of publication of application: 18.10.2006
(73) Proprietor: Kronosept AB, 241 23 Eslöv (SE)
(72) Inventor: EMOND, Anders, S-261 61 Landskrona (SE)
(86) International application number: PCT/SE2004/001643
(87) International publication number: WO 2005/044166

(56) References cited:
- EP-A2- 0 826 351
- WO-A1-91/07155
- WO-A1-96/20665
- FR-A1- 2 630 323
- SE-B- 426 206
- US-A- 3 863 637

## Description

Incontinence is a very common disease, app. 3 - 5 % of the population suffers from incontinence in one or another form. Several types of incontinence protection devices are present on the market. Most of these products are specially made for women but there are also products made for men.

This patent application describes a special embodiment of an absorbing incontinence device for men, but it can also be used by women, and gives a very effective protection against urine leakage.

Trough the unique design and accordion folding technique this incontinence device takes up a minimum of space in its folded state. It is also well suitable for individual packaging which makes it convenient for users to carry in their pockets.

### Prior art

In PCT WO 91/07155 an incontinence device for men is described. This product is due to its design very bulky and wide bodied, the form makes it clumsy and voluminous even after packaging.

The same goes for the incontinence device described in WO 96/05786.

Another type of incontinence device is described in US Pat.5,380,310 where the incontinence device is very long and narrow, folded twice, so called wing folding. The device is closed in its booth ends and is basically designed for women but can also be used by men.

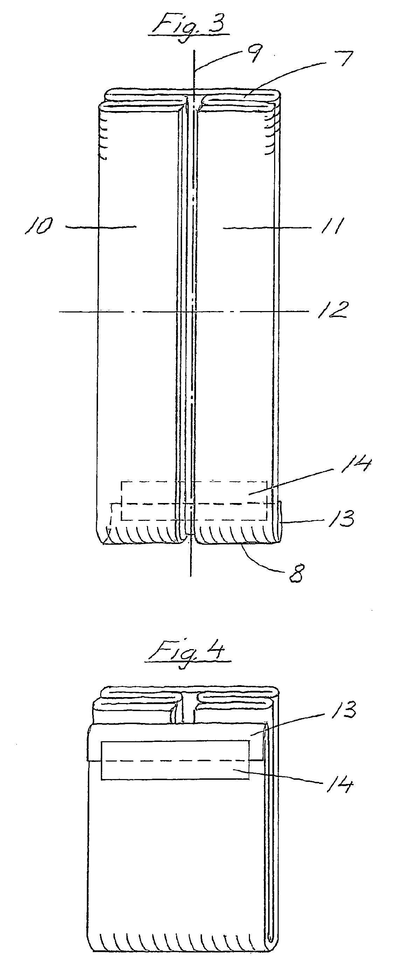

The Swedish patent SE7908400-0 describes another type of incontinence product especially designed for men. This product is folded twice in its longitudinal direction fig. 3 and in another embodiment fig. 4 in Z-folding configuration. None of these folds gives the advantage of the incontinence device described in this patent application.

### Description of figures

**Fig. 1** shows the invention applied on a user were 1 is the centre section of the device, 2 is one of the longitudinal folding lines, 3 scrotum and 4 the penis of the user, 16 elastic side lines and 17 the curved section of the device.
**Fig. 2** shows the incontinence device in a partly unfolded position, in this embodiment without the elastics. This view is seen from the side which faces the user, were 1 is the middle section, 2 one of the folding lines, 5 the outside of the device and 6 the lower section of the device forming a bowl when fully unfolded and 18 the sealed lower end of the device.
**Fig. 3** shows the device in its accordion folded state, were 7 is the upper end of the device and 8 the lower end. 9 shows a centre line in the longitudinal direction, 10 the accordion folded section at one side of the centre line 9 and 11 the accordion folded section on the other side of the centre line 9. 12 shows a transverse folding line in the centre of the device, 13 a flap, which is folded around the lower end of the device in order to make a closure and 14 a tape, which attaches the flap against the outside of the middle section.
**Fig. 4** shows the device folded in its transverse direction around the folding line 12 in fig. 3, 13 shows the flap which is folded around the lower end of the device, in order to make a closure and 14 a tape, which attaches the flap against the outside of the middle section.

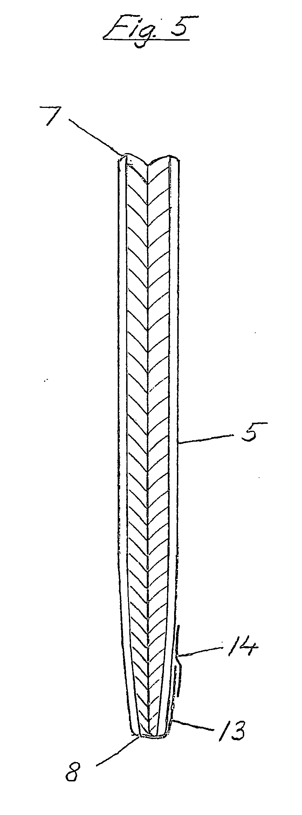

**Fig. 5** shows the incontinence device seen from the side with one of the accordion folded sections slightly unfolded. 7 shows the upper end and 8 the lower end of the device. 13 shows the flap folded around the lower end of the device, attached with a tape 14 against the outside of the middle section 5 of the device.
**Fig. 6** shows a cut away, of the device, along the line 12 in fig. 3, in an embodiment with three folds on each side of the centre line 9 in fig. 3.
**Fig. 7** shows a cut away, of the device, along the line 12 in fig. 3, in an embodiment with four folds on each side of the centre line 9 in fig. 3.
**Fig. 8** shows a cut away, of the device, along the line 12 in fig. 3, in an embodiment with five folds on each side of the centre line 9 in fig. 3.

### Description of the invention and its function

This patent application describes an absorbing incontinence protection device, preferably intended for men, principally made of an absorbent layer situated between an inner layer of liquid permeable material and an outer layer of a liquid impermeable material, folded in a unique way, which allows the device to take up a minimum of space in the consumer package. The layers are as a whole or partly connected to each other. The absorbent layer can be made of tissue, cellulose fluff, soft paper, airlaid cellulose, nonwoven or combinations thereof. The absorbent layer can, in different sections of the device, consist of one or more layers and the surface weight of such designs can vary, for example can the absorbent layer be of a higher surface weight in the centre and lower section of the device, than in the side sections. The absorbent layer can as a whole or partly contain superabsorbents with a weight concentration of between 1 - 85 %, for example the superabsorbent can be concentrated to the lower middle section of the device.

The average surface weight of the absorbent layer can vary depending on which grade of incontinence the device is intended for. If the incontinence device is intended for light dribble incontinence, it can be enough with a surface weight of the absorbent layer between 10-60 g/m2, for middle incontinence between 60 - 250 g/m2 and for more severe incontinence between 250 -1200 g/m2 or more.

Superabsorbents and absorbent layers of this kind are well known by man skilled in the art. The description above shows different kinds of absorbent layers, but other designs are possible and fits in the frame of this invention. Between the liquid permeable inner layer and the absorbent layer, can also an acquisition layer, a type of high loft nonwoven, be located in order to speed up the acquisition of the urine in the device and make the inner surface dryer. The incontinence device can also contain odour control additives and/or bacteria or fungicide growth inhibitors.

The outer layer 5, which is designed to prevent urine to pass to the outside of the incontinence device, is made of liquid impervious materials and can be made of plastic films, for example polyethylene, polypropylene, or other for the purpose suitable polymers. The liquid impervious outer layer can also be made of breathable plastic films with high water vapour permeability, preferably > 500 g/m2/24 hours at 38°C. The thickness of the films can be between 0,005 - 0,1 mm, preferably between 0,008 - 0,060 mm. The liquid impervious outer layer can also be made of hydrophobic nonwoven with a good liquid penetration resistance, for example between 4-100 cm water column, according to test method ERT 1201, preferably > 8 cm.

The liquid barrier can also be equipped with a nonwoven or tissue on its outside, in order to give it a more attractive non plastic look and to avoid direct contact between the users skin and the plastic film.

These types of liquid barriers are well known by the man skilled in the art.

The liquid permeable inner layer can be made of apertured plastic film, nonwoven or different types of woven fabrics as long as fluid easily can penetrate trough the layer. Common materials in this field are cotton, viscose, polypropylene, polyester, polyethylene and mixes thereof. A preferred embodiment is spunbonded nonwoven, made of polypropylene, with a surface weight between 10-50 g/m2, preferably 10-30 g/m2 surface weight.

The inner layer can be expelled, if the absorbent layer is of a quality comfortable enough to be in contact with the wearers skin. One embodiment of the device is to use a hydroentangled viscose nonwoven as a combined inner and absorbent layer.

The incontinence device is held in place by the underwear of the user, or preferably for incontinence especially developed elastic underwear. The outside of the device is preferably equipped with areas of self tacking adhesive, covered with release papers, which are to be removed before use. This adhesive is effectively sticking to the underwear, holding the device very effectively in place.

Other means of securing the device in place, is friction layers of different kinds, for example Velcro or expanded polymers as polyurethane foam.

These kinds of layers are well known by the person skilled in the art and are frequently used on sanitary napkins.

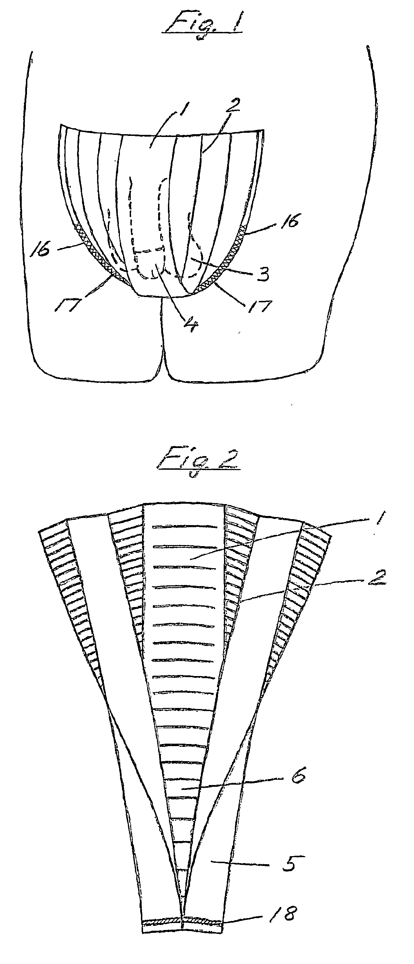

A further improvement of the device, can be reached by adding elastics to each side edge of the device. This will improve the bowl formation of the lower section. The elastics can be applied over the full length of the side edges or having an extension partly on the side edges as shown in fig. 1,16, for example only over the curved sections in fig. 1, 17. The elastic members can be made of latex or polymers or other elastic materials.

The application of the elastic members can be made by extending the inner and/or outer layer along the side edges of the device to apply the elastic member by gluing and folding. Methods of applying elastics to hygiene products are well known by the man skilled in the art.

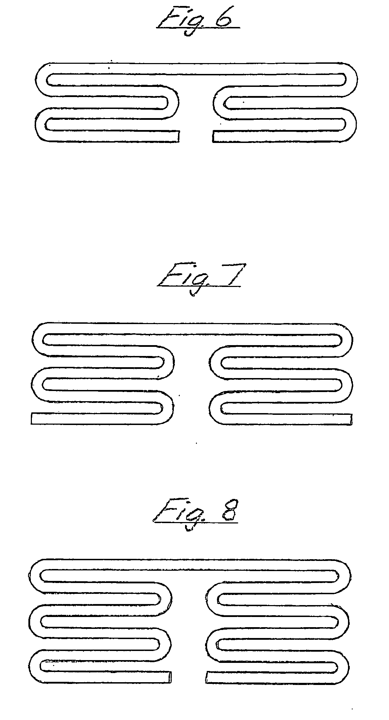

By making a number of folds in the longitudinal direction of the device, the space it requires in a consumer packing can be minimised. The device can be made with three folds as shown in fig. 6, or with four as shown in fig. 7, or with five as shown in fig. 8, or with more folds. The distance between the two accordion folded sections, when they are facing each other against the centre line 9 in the device, should preferably be as close as possible, but can vary from 0-50 mm, preferably 0-20 mm.

The folds are preferably of the same width, but can also be allowed to vary, for example the first fold can be wider than the next fold, which in its turn can be narrower then the following fold and so on. Other variations and configurations of the folds can be possible within the scope of this invention. The width of the accordion folded sections can vary from 5-100 mm, preferably 10-50 mm, which gives the device a total width of 15 - 205 mm or 25-105 mm, if the distance between the accordion folded sections, when they are facing each other against the centre line 9, is 5 mm. With a configuration of three folds as shown in fig. 3 and fig. 4, the maximum width of an unfolded device, when the width of the folds are 20 mm and the distance between the accordion folded sections is 5 mm, will be 165 mm.

The incontinence device can also with advantage be folded in its transverse direction one or several times, to further reduce its size in a consumer package. Fig. 4 shows an example, where the device in fig. 3 has been folded once in its transverse direction along the centre line 12 in fig. 3.

One preferred embodiment of this invention is to individually pack the devices.

The individual package material can for example be made of plastic film, tissue paper, nonwoven or combinations hereof and be formed as a pouch surrounding the device. This type of individually packaging is well known by the man skilled in the art and is often used to pack panty liners and sanitary napkins.

The lower end 8 of the device, is preferably closed, after the accordion folds are made, where the outer and inner layers at the lower end of the device are extended beyond the absorbent layer, to form a flap 13, which is folded backwards 180° and fastened to the outer layer of the centre section 5, preferably with a self adhesive tape 14 or with glue, hot melt adhesive, heat seal, ultrasonic, mechanical fastening or combinations thereof, or in other suitable manners in order to achieve a sufficient closure of the end section of the device. The closure of the lower end of the device can also be achieved by making a flap of either the outer or inner layer to be fastened to the outer layer of the centre section, in the same way as described for the outer and inner layer together. The closure of the lower end of the device can also be achieved by folding all the layers of the device, including the absorbent layer, and fasten to the outside of the device in the same manner as described above. The length of the folded flap can be anything from 3-100 mm, preferably 5-50 mm.

Another way to close the lower end is to seal the flap outside of the absorbent layer, fig. 2, 18 without folding it. The outer or inner layers can be extended beyond the absorbent layer and sealed or both the inner and outer layers together can be extended outside of the absorbent layer and be sealed or all the layers of the device, including the absorbent layer, can be sealed together. The sealing can be made with for example, tape, heat sealing, hotmelt adhesive or by other means strong enough to keep the layers together, so they do not break apart when the device is in use. The lower end can also be closed by the folding of a tape all around the end section of the device.

### Handling and application of the incontinence device

Before use the incontinence device is unfolded. Since the device is closed in its lower end it will form a bowl in its lower section fig. 1 and fig. 2. The device should then be placed in front of the penis with the bowl formed lower section under the penis or under the penis and scrotum, whatever is preferred by the user. The side edges should then be pushed up against the groins of the user.

If the device is equipped with self tacking adhesive, the protective release papers are removed and the device is fastened to the underwear.

### Advantage with the invention

Trough the inventions unique accordion folding technique, a rather large incontinence device can be brought down in size to take a minimum of space in a consumer package.

This is of very big importance for the user, who discretely can bring one or more products with him, especially if they are individually packed, in for example, the breast pocket or other pockets in his clothing. Since incontinence for many persons are burdened with guilt, this gives the incontinent person an improved confidence and improves his social situation. When the incontinence device, according to the invention, is used in institutions or long term wards, the reduced space makes it much easier to store. The incontinence device is also much easier to bring around on service trolleys with limited space available.

### Example of design

Incontinence protection device made of an outer layer of polyethylene film, with a thickness of 0,02 mm, and an inner layer of spunbonded polypropylene nonwoven, with a surface weight of 23 g/m2. Located between these two layers is an absorbent layer. The absorbent layer is made of 63% cellulose, 25% superabsorbent, 7 % synthetic fibres and 5% latex, the surface weight is 150 g/m2 (for example Concert GmbH GH.150-1008).

The layers are joined together with hot melt adhesive. The absorbent layer has the shape of a rectangle with the dimension of 180 x 230 mm. The outer and inner layers have a somewhat larger areas than the absorbent layer, with a width of 190 mm and a length of 270 mm. These layers are located on each side of the absorbent layer and situated so that the side edges extend 5 mm on each side, the upper edge extends 10 mm and the lower edge 30 mm, outside the absorbent layer.

The layers are folded three times on each side in the longitudinal direction, as shown in fig. 3 and fig. 6, which gives the device an accordion folded configuration.

The fold width is 20 mm for each single fold, and the distance between the accordion folded sections, in the centre of the device, is 5 mm, which gives the device a total width of 45 mm. The flap 13, fig. 3, which sticks out 30 mm from the absorbent layer is folded backwards up against the outer layer and sealed to it with a self tacking tape covering the whole width, 45 mm, of the accordion folded device. The device is then folded once in the centre, in its transverse direction, which gives the device a size of 45 x 125 mm, when the upper flap of 10 mm is included.

The device has a self adhesive layer on the outside of the outer layer, located in the centre of the centre section 1 of the device. The width of the adhesive layer is 10 mm and has a longitudinal extension of 170 mm, leaving an uncovered space at the bottom of the device of 40 mm and at the top of 20 mm. The adhesive is covered with a siliconized release paper.

## Claims

1. Incontinence protection device principally made of a liquid impermeable outer layer, a liquid permeable inner layer and an absorbent layer located between these layers **characterised in that** the device is accordion folded, in its longitudinal direction, with three folds or more on each side of the centre line (9) of the device, forming two mirror reversed accordion folded sections (10, 11), one on each side of the centre line (9), the lower end of the device is closed, which gives it a bowl formed lower section (6) when unfolded.

2. Incontinence protection device according to claim 1 **characterised in that** the device is closed in its lower end, by the extension of the inner or outer layer or inner and outer layer together outside the absorbent layer, forming a flap (13), which is folded forward or backward 180° and fastened to the outer layer of the device.

3. Incontinence protection device according to claim 1 **characterised in that** the device is closed in its lower end, by folding all layers including the absorbent layer backwards or forwards 180° and fasten them to the outer layer of the device.

4. Incontinence protection device according to claim 1 **characterised in that in that** the device is closed in its lower end, by the extension of the inner or outer layer or inner and outer layer together outside the absorbent layer, forming a flap, where the layers are sealed together.

5. Incontinence protection device according to claim 1 **characterised in that** the device is closed in its lower end, by the folding of a tape around the lower section of the device or by sealing the accordion folded sections together.

6. Incontinence protection device according to claim 1 **characterised in that** the device is equipped with elastics (16) partly or as a whole along its side edges.

7. Incontinence protection device according to claim 1-6 **characterised in that** the device is folded one or several times in its transverse direction.

8. Incontinence protection device according to claim 1-7 **characterised in that** the device is individually packed.

## Patentansprüche

1. Inkontinenzschutzvorrichtung, die hauptsächlich aus einer flüssigkeitsundurchlässigen Außenschicht, einer flüssigkeitsdurchlässigen Innenschicht und einer zwischen diesen Schichten angeordneten absorbierenden Schicht besteht, **dadurch gekennzeichnet, dass** die Vorrichtung in ihrer Längsrichtung mit drei oder mehr Falten auf jeder Seite der Mittellinie (9) der Vorrichtung wie ein Akkordeon gefaltet ist, wobei die Falten auf jeder Seite der Mittellinie (9) zwei spiegelbildliche, wie ein Akkordeon gefaltete Abschnitte (10, 11) bilden, wobei das untere Ende der Vorrichtung geschlossen ist, was in aufgefaltetem Zustand einen schüsselförmigen unteren Abschnitt (6) ergibt.

2. Inkontinenzschutzvorrichtung nach Anspruch 1, **dadurch gekennzeichnet, dass** die Vorrichtung durch die Erweiterung der inneren oder der äußeren Schicht oder der inneren und der äußeren Schicht gemeinsam über die absorbierende Schicht hinaus, die eine Klappe (13) bildet, welche um 180° nach vorn oder hinten umgefaltet und an der Außenschicht der Vorrichtung befestigt ist, an ihrem unteren Ende geschlossen ist.

3. Inkontinenzschutzvorrichtung nach Anspruch 1, **dadurch gekennzeichnet, dass** die Vorrichtung durch Umfalten aller Schichten, einschließlich der absorbierenden Schicht, um 180° nach hinten oder vorn und Befestigen derselben an der Außenschicht der Vorrichtung an ihrem unteren Ende geschlossen ist.

4. Inkontinenzschutzvorrichtung nach Anspruch 1, **dadurch gekennzeichnet, dass** die Vorrichtung durch die Erweiterung der inneren oder der äußeren Schicht oder der inneren und der äußeren Schicht gemeinsam über die absorbierende Schicht hinaus, die eine Klappe bildet, an ihrem unteren Ende geschlossen ist, wobei die Schichten miteinander verbunden sind.

5. Inkontinenzschutzvorrichtung nach Anspruch 1, **dadurch gekennzeichnet, dass** die Vorrichtung durch das Falten eines Bandes um den unteren Abschnitt der Vorrichtung herum oder durch Versiegeln der wie ein Akkordeon gefalteten Abschnitte miteinander an ihrem unteren Ende geschlossen ist.

6. Inkontinenzschutzvorrichtung nach Anspruch 1, **dadurch gekennzeichnet, dass** die Vorrichtung entlang ihrer Seitenränder teilweise oder vollständig mit Gummibändern (16) ausgestattet ist.

7. Inkontinenzschutzvorrichtung nach Anspruch 1 bis 6, **dadurch gekennzeichnet, dass** die Vorrichtung in ihrer Querrichtung einmal oder mehrere Male gefaltet ist.

8. Inkontinenzschutzvorrichtung nach Anspruch 1 bis 7, **dadurch gekennzeichnet, dass** die Vorrichtung einzeln verpackt ist.

## Revendications

1. Dispositif de protection pour incontinence fait principalement d'une couche extérieure imperméable au liquide, d'une couche intérieure perméable au liquide et d'une couche absorbante placée entre ces couches, **caractérisé en ce que** le dispositif est plié en accordéon dans son sens longitudinal, avec trois plis ou plus de chaque côté de la ligne centrale (9) du dispositif, ce qui forme deux sections pliées en accordéon inversées en miroir (10, 11), une de chaque côté de la ligne centrale (9), l'extrémité inférieure du dispositif étant fermée, ce qui donne une section inférieure en forme de bol (6) une fois déplié.

2. Dispositif de protection pour incontinence selon la revendication 1, **caractérisé en ce que** le dispositif est fermé à son extrémité inférieure par l'extension de la couche intérieure ou extérieure ou intérieure et extérieure ensemble à l'extérieur de la couche absorbante, ce qui forme un rabat (13) qui est replié vers l'avant ou vers l'arrière de 180° et fixé à la couche extérieure du dispositif.

3. Dispositif de protection pour incontinence selon la revendication 1, **caractérisé en ce que** le dispositif est fermé à son extrémité inférieure en repliant toutes les couches, compris la couche absorbante, vers l'arrière ou vers l'avant de 180° et en les fixant sur la couche extérieure du dispositif.

4. Dispositif de protection pour incontinence selon la revendication 1, **caractérisé en ce que** le dispositif est fermé à son extrémité inférieure par l'extension de la couche intérieure ou extérieure ou intérieure et extérieure ensemble à l'extérieur de la couche absorbante, ce qui forme un rabat où les couches sont scellées ensemble.

5. Dispositif de protection pour incontinence selon la revendication 1, **caractérisé en ce que** le dispositif est fermé à son extrémité inférieure en pliant une bande autour de la section inférieure du dispositif ou en scellant les sections pliées en accordéon ensemble.

6. Dispositif de protection pour incontinence selon la revendication 1, **caractérisé en ce que** le dispositif est équipé partiellement ou intégralement d'élastiques (16) le long de ses bords latéraux.

7. Dispositif de protection pour incontinence selon les revendications 1 à 6, **caractérisé en ce que** le dispositif est plié une ou plusieurs fois dans son sens transversal.

8. Dispositif de protection pour incontinence selon les revendications 1 à 7, **caractérisé en ce que** le dispositif est emballé individuellement.
